# EUROPEAN PATENT SPECIFICATION

(11) **EP 0 952 808 B1**
(45) Date of publication and mention of the grant of the patent: **22.08.2001**
(21) Application number: 97952506.0
(22) Date of filing: 17.12.1997
(51) Int. Cl.: A61K 7/00

(54) **PACKAGED PERSONAL CLEANSING PRODUCT**
VERPACKTES KOERPERREINIGUNGSMITTEL
PRODUIT DE NETTOYAGE PERSONNEL CONDITIONNE

(30) Priority: 20.12.1996 GB 9626553; 09.04.1997 GB 9707244
(43) Date of publication of application: 03.11.1999
(73) Proprietor: THE PROCTER & GAMBLE COMPANY, Cincinnati, Ohio 45202 (US)
(72) Inventor: DAWSON, Geoffrey, George, Virginia Water Surrey GU25 4EU (GB); BELLEMAIN, Chantal, Marie-Claude, Egham, Surrey TW20 0JH (GB)
(74) Representative: Woof, Victoria
(86) International application number: US9723335
(87) International publication number: WO9827937

(56) References cited:
- EP-A- 0 213 827
- EP-A- 0 677 577
- WO-A-92/06669
- WO-A-93/21293
- GB-A- 2 290 551
- J. MUFTI: "the aerosol mousse" COSMETICS & TOILETRIES, vol. 110, June 1995, pages 50-53, XP002062568

## Description

The present invention relates to a packaged personal cleansing product comprising a foaming composition and a propellant gas packaged within a container. In particular, the present invention relates to a packaged personal cleansing product which provides improved deposition of skin/hair care ingredients, excellent lather properties and an increase in the level and type of skin/hair care ingredients which can be delivered, together with increased perfume delivery and residuality.

### Background of the Invention

Personal cleansing compositions must satisfy a number of criteria including cleansing power, foaming properties, mildness/low irritancy and good feel with respect to the skin, hair and the ocular mucosae. Ideal personal cleansers should also provide good deposition of skin/hair care ingredients such as, for example, oils, conditioning agents, anti-oxidants, vitamins, antibacterials, UV sunscreens, tanning agents, exfoliants, warming and cooling agents. Ideal personal cleansing compositions should also provide good perfume delivery and residuality to the skin and hair. However, these benefits can be difficult to achieve and most lathering soaps, shower and bath products, shampoos and bars fail in these respects.

It can be difficult to provide a personal cleansing composition having both low skin irritation and high lathering benefits. This is because surfactants which enhance lather (e.g. alkyl sulfates) tend to be relatively harsh to the skin and surfactants which are mild, tend to produce inferior lather. It can also be difficult to provide a personal cleansing composition having good deposition and efficacy of skin care ingredients such as oils, vitamins and UV screens. This is because many such skin care ingredients are water-insoluble and/or unstable to moisture, air or light and either cannot be easily and stably incorporated into conventional aqueous compositions, or must be incorporated as emulsions which then have limited deposition.

Thus a need exists for personal cleansing products which provide improved deposition of skin/hair care ingredients and which exhibit improved product stability, at the same time as providing excellent cleansing, lathering, mildness and increased skin/hair care ingredient deposition, as well as improved perfume delivery and residuality.

WO 92/11839 (L. Mackles; published 23 July 1992) discloses an anhydrous topically applicable aerosol foam composition comprising a foamable anhydrous liquid, a foaming agent selected from methyl glucose C₁₆-C₁₈ aliphatic acid esters and a propellant. The level of foaming agent is from 0.2 to 10% by weight of composition.

US-677577 discloses compositions comprising different combinations of the features water content, surfactant concentration and viscosity.

It has now surprisingly been found that by providing a packaged personal cleansing product comprising a foaming composition comprising 25% or less of water, 18% or more of surfactant and from 1% to 50% by weight of a water-insoluble skin or hair care ingredient, and a propellant gas packaged within a container, a product is provided which exhibits excellent lather and mildness, improved deposition of skin/hair care ingredients and improved perfume delivery and residuality.

### Summary of the Invention

According to the present invention there is provided a packaged personal cleansing product comprising:
(i) a foaming composition, wherein the foaming composition comprises less than 25% by weight, preferably less than 15% by weight, of water, at least 18% by weight of surfactant, said surfactant being selected from anionic, nonionic, cationic, zwitterionic and amphoteric surfactants and mixtures thereof, and from 1% to 50% by weight of a water-insoluble skin or hair care ingredient;
(ii) propellant gas; and
(iii) a container for (i) and (ii);
characterised in that the propellant gas comprises carbon dioxide, nitrous oxides, or mixtures thereof and that the viscosity of the foaming composition, measured at 20°C and 1 atmosphere, using a Brookfield viscometer, No.2 Spindle at 60 rpm, is less than 700 mPa.s.

The packaged personal cleansing product of the present invention provides improved lathering, mildness, improved deposition of skin/hair care ingredients onto the skin and improved perfume delivery and residuality.

### Detailed Description of the Invention

The packaged product of the present invention comprises a foaming composition, a propellant and a container.

### Foaming composition

The foam composition herein comprises less than 25% by weight, preferably less than 15% by weight, more preferably less than 5% by weight of water.

The foam composition herein comprises at least 18% by weight of surfactant selected from anionic, nonionic, cationic, zwitterionic and amphoteric surfactants and mixtures thereof.

Mild surfactants suitable for inclusion in compositions according to the present invention generally have a lipophilic chain length of from 8 to 22 carbon atoms and can be selected from anionic, nonionic, zwitterionic and amphoteric surfactants and mixtures thereof. The total level of surfactant is preferably from 20% to 80%, more preferably from 25% to 65%, and especially from 30% to 50% by weight. The compositions may comprise a mixture of anionic with zwitterionic and/or amphoteric surfactants. The level of the anionic surfactant is in the range from 1% to 70%, and especially from 10% to 50% by weight of the composition, while the level of nonionic surfactant, where present, is in the range from 2% to 25% by weight, preferably from 5% to 20% by weight. The weight ratio of anionic surfactant: zwitterionic and/or amphoteric surfactant is in the range from 10:1 to 0.5:1, preferably from 7:1 to about 1:1, more preferably from 4:1 to 1:1.

Anionic surfactants suitable for inclusion in the compositions of the invention can generally be described as mild synthetic detergent surfactants and include alkyl sulphates, ethoxylated alkyl sulfates, alkyl glyceryl ether sulfonates, methyl acyl taurates, fatty acyl glycinates, N-acyl glutamates, acyl isethionates, acyl ethoxylated isethionates, acyl short chain branched isethionates, fatty amido ethoxylated carboxylates, alkyl sulfosuccinates, alkyl ethoxysulphosuccinates, alpha-sulfonated fatty acids, their salts and/or their esters, alkyl phosphate esters, ethoxylated alkyl phosphate esters, acyl sarcosinates and fatty acid/protein condensates, paraffin sulphonates, alpha-olefin sulphonates, and mixtures thereof. Alkyl and/or acyl chain lengths for these surfactants are C₁₂-C₂₂, preferably C₁₂-C₁₈ more preferably C₁₂-C₁₄.

Surfactants of this class also include short-chain alkyl sulphate surfactants where 'short chain' as defined herein means an average carbon chain length of C₁₀ or less. The short chain alkyl sulphate surfactants of the present invention are valuable in shower gel compositions for the delivery of improved skin mildness attributes and product rinsing benefits in combination with a desirable lather profile. Alkyl sulphate surfactants suitable for inclusion in the compositions of the present invention have the general formula (II);

R - SO₃ - M

wherein R is straight or branched chain alkyl, preferably straight chain, containing on average from 8 to 10 carbon atoms, preferably 10 carbon atoms and wherein M is selected from alkali metals, alkaline earths, alkyl ammonium or alkanol ammonium, ammonium or other suitable monovalent cation or mixtures thereof, preferably monoethanolamine. It should be understood that the definition of any particular carbon chain length, say C₈ is an average value and as such may contain certain proportions of both higher and lower carbon chain lengths as a direct function of its synthesis. The level of such material can be achieved by modification of the process and the nature of the starting materials. While C₁₀ alkyl sulphate is the preferred surfactant in the compositions of the invention mixtures of short chain alkyl sulphates may also be used. Especially preferred in the compositions herein is C₁₀ alkyl sulphate material containing at least about 80% by weight of the C₁₀, preferably at least 90% C₁₀, more preferably at least 95% C₁₀ and especially at least 99% C₁₀ alkyl sulphate.

Additional anionic surfactants suitable for use in the compositions according to the present invention are the salts of sulfuric acid esters of the reaction product of 1 mole of a higher fatty alcohol and from 1 to 12 moles of ethylene oxide. The counterions can be alkali metal, alkaline earth metal, ammonium, mono-, di-, or trialkylammonium, mono-, di-, or tri-alkanol ammonium, with monoethanolamine being the preferred counterion. Particularly preferred are the alkyl ethoxy sulphates containing from 2 to 6, preferably 2 to 4 moles of ethylene oxide, such as sodium laureth-2 sulphate, and sodium laureth-3 sulphate. In preferred embodiments, the anionic surfactant contains at least 50% especially at least 75% by weight of ethoxylated alkyl sulphate.

In addition to the broad range ethoxylated alkyl sulphates obtained via conventional sodium catalysed ethoxylation techniques and subsequent sulphation processes, ethoxylated alkyl sulphates obtained from narrow range ethoxylates (NREs) are also suitable anionic surfactants for use in the present compositions. Narrow range ethoxylated alkyl sulphates suitable for use herein are selected from sulphated alkyl ethoxylates containing on average from 1 to 6, preferably from 2 to 4 and especially 3 moles of ethylene oxide such as NRE laureth-3 sulphate. NRE materials suitable for use herein contain distributions of the desired ethylene oxide (EOn) in the ranges of from 15% to 30% by weight of EOₙ, from 10% to 20% by weight of EOₙ₊₁ and from 10% to 20% by weight of EOₙ₋₁. Highly preferred NRE materials contain less than 9% by weight of ethoxylated alkyl sulphate having 7 or more moles of ethylene oxide and less than 13% by weight of non-ethoxylated alkyl sulphate.

The compositions according to the present invention may comprise nonionic surfactant at levels from 2% to 25%, more preferably from 5% to 20% by weight. Surfactants of this class include C₁₂-C₁₈ fatty acid mono-and dialkanolamides such as cocoethanolamide, cocomonoisopropylamide, cocodiethanolamide and ethoxylated derivatives thereof, alkyl poly glucosides, C8-C22 fatty alcohol ethoxylates with 1 to 10 ethoxylate groups, sucrose polyester surfactants and polyhydroxy fatty acid amide surfactants having the general formula (III).

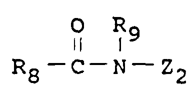

The preferred N-alkyl, N-alkoxy or N-aryloxy, polyhydroxy fatty acid amide surfactants according to formula (III) are those in which R₈ is C₅-C₃₁ hydrocarbyl, preferably C₆-C₁₉ hydrocarbyl, including straight-chain and branched chain alkyl and alkenyl, or mixtures thereof and R₉ is typically hydrogen, C₁-C₈ alkyl or hydroxyalkyl, preferably methyl, or a group of formula -R¹-O-R² wherein R¹ is C₂-C₈ hydrocarbyl including straight-chain, branched-chain and cyclic (including aryl), and is preferably C₂-C₄ alkylene, R² is C₁-C₈ straight-chain, branched-chain and cyclic hydrocarbyl including aryl and oxyhydrocarbyl, and is preferably C₁-C₄ alkyl, especially methyl, or phenyl. Z₂ is a polyhydroxyhydrocarbyl moiety having a linear hydrocarbyl chain with at least 2 (in the case of glyceraldehyde) or at least 3 hydroxyls (in the case of other reducing sugars) directly connected to the chain, or an alkoxylated derivative (preferably ethoxylated or propoxylated) thereof. Z₂ preferably will be derived from a reducing sugar in a reductive ammination reaction, most preferably Z₂ is a glycityl moiety. Suitable reducing sugars include glucose, fructose, maltose, lactose, galactose, mannose, and xylose, as well as glyceraldehyde. As raw materials, high dextrose corn syrup, high fructose corn syrup, and high maltose corn syrup can be utilised as well as the individual sugars listed above. These corn syrups may yield a mix of sugar components for Z₂. It should be understood that it is by no means intended to exclude other suitable raw materials. Z₂ preferably will be selected from the group consisting of -CH₂-(CHOH)ₙ-CH₂OH, -CH(CH₂OH)-(CHOH)ₙ₋₁-CH₂H, CH₂(CHOH)₂(CHOR')CHOH)-CH₂OH, where n is an integer from 1 to 5, inclusive, and R' is H or a cyclic mono- or poly-saccharide, and alkoxylated derivatives thereof. As noted, most preferred are glycityls wherein n is 4, particularly -CH₂-(CHOH)₄-CH₂OH.

The most preferred polyhydroxy fatty acid amide has the formula R₈(CO)N(CH₃)CH₂(CHOH)₄CH₂OH wherein R₈ is a C₆-C₁₉ straight chain alkyl or alkenyl group. In compounds of the above formula, R₈-CO-N< can be, for example, cocoamide, stearamide, oleamide, lauramide, myristamide, capricamide, palmiamide, tallowamide, etc.

A process for making the above compounds having formula (III) comprises reacting a fatty acid triglyceride or a fatty methyl ester with an N-substituted polyhydroxy amine in the substantial absence of lower (C₁-C₄) alcoholic solvent, but preferably with an alkoxylated alcohol or alkoxylated alkyl phenol such as NEODOL and using an alkoxide catalyst at temperatures of from about 50°C to about 140°C to provide high yields (90-98%) of the desired products. Suitable processes for making the desired polyhydroxy fatty acid amide compounds are outlined in US-A-5,194,639 and US-A-5,380,891.

The compositions for use herein may also contain an amphoteric surfactant at a level of from 2% to 25%, preferably from 5% to 15%, by weight. Amphoteric surfactants suitable for use in the compositions of the invention include:
(a) imidazolinium surfactants of formula (IV) 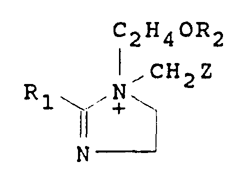 wherein R₁ is C₇-C₂₂ alkyl or alkenyl, R₂ is hydrogen or CH₂Z, each Z is independently CO₂M or CH₂CO₂M, and M is H, alkali metal, alkaline earth metal, ammonium or alkanolammonium; and/or ammonium derivatives of formula (V) 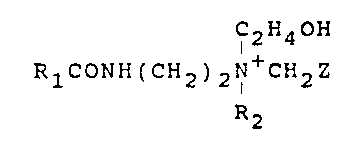 wherein R₁, R₂ and Z are as defined above;
(b) aminoalkanoates of formula (VI)

   R₁NH(CH₂)ₙCO₂M

   iminodialkanoates of formula (VII)

   R₁N[(CH₂)ₘCO₂M]₂

   and iminopolyalkanoates of formula (VIII) 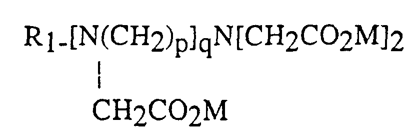 wherein n, m, p, and q are numbers from 1 to 4, and R₁ and M are independently selected from the groups specified above; and
(c) mixtures thereof.

Examples of suitable amphoteric surfactants of type (a) include compounds of formula IV and/or V in which R₁ is C₈H₁₇ (especially iso-capryl), C₉H₁₉ and C₁₁H₂₃ alkyl. Especially preferred are the compounds in which R₁ is C₉H₁₉, Z is CO₂M and R₂ is H; the compounds in which R₁ is C₁₁H₂₃, Z is CO₂M and R₂ is CH₂CO₂M; and the compounds in which R₁ is C₁₁H₂₃, Z is CO₂M and R₂ is H.

In CTFA nomenclature, materials suitable for use in the present invention include cocoamphocarboxypropionate, cocoamphocarboxy propionic acid, and especially cocoamphoacetate and cocoamphodiacetate (otherwise referred to as cocoamphocarboxyglycinate).

The compositions herein can also contain from 2% to 30%, more preferably from 5% to 15% of a zwitterionic surfactant.

Betaine surfactants suitable for inclusion in the compositions of the present invention include alkyl betaines of the formula R₅R₆R₇N⁺ (CH₂)ₙCO₂M and amido betaines of the formula (IX) 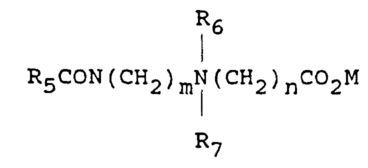 wherein R₅ is C₁₁-C₂₂ alkyl or alkenyl, R₆ and R₇ are independently C₁-C₃ alkyl, M is H, alkali metal, alkaline earth metal, ammonium or alkanolammonium, and n, m are each numbers from 1 to 4. Preferred betaines include cocoamidopropyldimethylcarboxymethyl betaine, laurylamidopropyldimethylcarboxymethyl betaine and Tego betaine (RTM).

Sultaine surfactants suitable for inclusion in the compositions of the present invention include alkylamido sultaines of the formula; 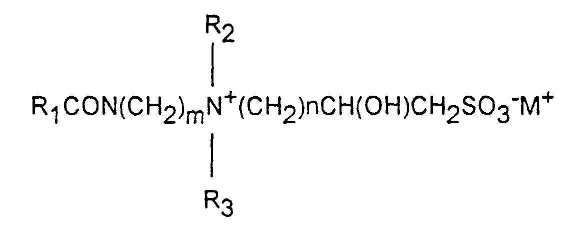 wherein R₁ is C₇ to C₂₂ alkyl or alkenyl, R₂ and R₃ are independently C₁ to C₃ alkyl, M is H, alkali metal, alkaline earth metal, ammonium or alkanolammonium and m and n are numbers from 1 to 4. Preferred for use herein is coco amido propylhydroxy sultaine.

Amine oxide surfactants suitable for inclusion in the compositions of the present invention include alkyl amine oxide R₅R₆R₇NO and amido amine oxides of the formula: 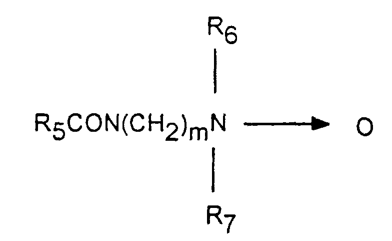 wherein R₅ is C₁₁ to C₂₂ alkyl or alkenyl, R₆ and R₇ are independently C₁ to C₃ alkyl, M is H, alkali metal, alkaline earth metal, ammonium or alkyl ammonium, alkanolammonium and m is a number from 1 to 4. Preferred amine oxides include cocoamidopropylamine oxide, lauryl dimethyl amine oxide and myristyl dimethyl amine oxide.

The foaming composition herein also comprises from 1% to 50%, preferably from 5% to 50%, more preferably from 15% to 40%, by weight, of a water-insoluble skin or hair care ingredient.

Addition of such water-insoluble skin or hair care ingredients can provide emolliency, mildness and rinsibility characteristics to personal cleansing compositions according to the invention.

Suitable insoluble skin or hair care ingredients for use herein can be selected from water-insoluble silicones inclusive of non-volatile polyalkyl and polyaryl siloxane gums and fluids, volatile cyclic and linear polyalkylsiloxanes, polyalkoxylated silicones, amino and quaternary ammonium modified silicones, rigid cross-linked and reinforced silicones and mixtures thereof, C₁-C₂₄ esters of C₈-C₃₀ fatty acids such as isopropyl myristate, myristyl myristate and cetyl ricinoleate, C₈-C₃₀ esters of benzoic acid, beeswax, saturated and unsaturated fatty alcohols such as behenyl alcohol, hydrocarbons such as mineral oils, petrolatum squalane and squalene, polybutene, polyalphaolefins such as polydecene, fatty sorbitan esters (see US-A-3988255, Seiden, issued October 26th 1976), lanolin and oil-like lanolin derivatives, animal and vegetable triglycerides such as almond oil, peanut oil, wheat germ oil, rice bran oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soyabean oil, avocado oil, safflower oil, coconut oil, hazelnut oil, olive oil, grapeseed oil, and sunflower seed oil, and C₁-C₂₄ esters of dimer and trimer acids such as diisopropyl dimerate, diisostearylmalate, diisostearyldimerate and triisostearyltrimerate.

Another water-insoluble, skin/hair care ingredient suitable for use in the foaming compositions herein is a liquid, polyol carboxylic acid ester.

The polyol ester preferred for use herein is a nonocclusive liquid or liquifiable polyol carboxylic acid ester. These polyol esters are derived from a polyol radical or moiety and one or more carboxylic acid radicals or moieties. In other words, these esters contain a moiety derived from a polyol and one or more moieties derived from a carboxylic acid. These carboxylic acid esters can also be derived from a carboxylic acid. These carboxylic acid esters can also be described as liquid polyol fatty acid esters, because the terms carboxylic acid and fatty acid are often used interchangeably by those skilled in the art.

The preferred liquid polyol polyesters employed in this invention comprise certain polyols, especially sugars or sugar alcohols, esterified with at least four fatty acid groups. Accordingly, the polyol starting material must have at least four esterifiable hydroxyl groups.
Examples of preferred polyols are sugars, including monosaccharaides and disaccharides, and sugar alcohols. Examples of monosaccharides containing four hydroxyl groups are xylose and arabinose and the sugar alcohol derived from xylose, which has five hydroxyl groups, i.e., xylitol. The monosaccharide, erythrose, is not suitable in the practice of this invention since it only contains three hydroxyl groups, but the sugar alcohol derived from erythrose, i.e., erythritol, contains four hydroxyl groups and accordingly can be used. Suitable five hydroxyl group-containing monosaccharides are galactose, fructose, and sorbose. Sugar alcohols containing six -OH groups derived from the hydrolysis products of sucrose, as well as glucose and sorbose, e.g., sorbitol, are also suitable. Examples of disaccharide polyols which can be used include maltose, lactose, and sucrose, all of which contain eight hydroxyl groups.

Preferred polyols for preparing the polyesters for use in the present invention are selected from the group consisting of erythritol, xylitol, sorbitol, glucose, and sucrose. Sucrose is especially preferred.

The polyol starting material having at least four hydroxyl groups is esterified on at least four of the -OH groups with a fatty acid containing from 8 to 22 carbon atoms. Examples of such fatty acids include caprylic, capric, lauric, myristic, myristoleic, palmitic, palmitoleic, stearic, oleic, ricinoleic, linoleic, linolenic, eleostearic, arachidic, arachidonic, behenic, and erucic acid. The fatty acids can be derived from naturally occurring or synthetic fatty acids; they can be saturated or unsaturated, including positional and geometrical isomers. However, in order to provide liquid polyesters preferred for use herein, at least 50% by weight of the fatty acid incorporated into the polyester molecule should be unsaturated. Oleic and linoleic acids, and mixtures thereof, are especially preferred.

The polyol fatty acid polyesters useful in this invention should contain at least four fatty acid ester groups. It is not necessary that all of the hydroxyl groups of the polyol be esterified with fatty acid, but it is preferable that the polyester contain no more than two unesterified hydroxyl groups. Most preferably, substantially all of the hydroxyl groups of the polyol are esterified with fatty acid, i.e., the polyol moiety is substantially completely esterified. The fatty acids esterified to the polyol molecule can be the same or mixed, but as noted above, a substantial amount of the unsaturated acid ester groups must be present to provide liquidity.

To illustrate the above points, a sucrose fatty triester would not be suitable for use herein because it does not contain the required four fatty acid ester groups. A sucrose tetra-fatty acid ester would be suitable, but is not preferred because it has more than two unesterified hydroxyl groups. A sucrose hexa-fatty acid ester would be preferred because it has no more than two unesterified hydroxyl groups. Highly preferred compounds in which all the hydroxyl groups are esterified with fatty acids include the liquid sucrose octa-substituted fatty acid esters.

The following are non-limiting examples of specific polyol fatty acid polyesters containing at least four fatty acid ester groups suitable for use in the present invention: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof.

As noted above, highly preferred polyol fatty acid esters are those wherein the fatty acids contain from 14 to 18 carbon atoms.

The preferred liquid polyol polyesters preferred for use herein have complete melting points below 30°C, preferably below 27.5°C, more preferably below 25°C. Complete melting points reported herein are measured by Differential Scanning Calorimetry (DSC).

The polyol fatty acid polyesters suitable for use herein can be prepared by a variety of methods well known to those skilled in the art. These methods include: transesterification of the polyol with methyl, ethyl or glycerol fatty acid esters using a variety of catalysts; acylation of the polyol with a fatty acid chloride; acylation of the polyol with a fatty acid anhydride; and acylation of the polyol with a fatty acid, per se. See U.S. Patent No. 2,831,854; U.S. Patent No. 4,005,196, to Jandacek, issued January 25, 1977; U.S. Patent No. 4,005,196, to Jandacek, issued January 25, 1977.

A further water-insoluble, skin/hair care ingredient suitable for use herein is an emollient material selected from compounds having the formula (I): 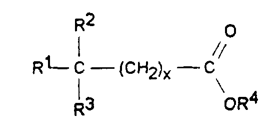 wherein R¹ is selected from H or CH₃, R², R³ and R⁴ are independently selected from C₁-C₂₀ straight chain or branched chain alkyl, and x is an integer of from 1-20,
and compounds having the formula (II): 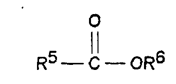
wherein R⁵ is selected from optionally hydroxy or C₁-C₄ alkyl substituted
benzyl and R₆ is selected from C₁-C₂₀ branched or straight chain alkyl; and
mixtures thereof.

Preferred for use herein include emollients having the general formula (I) and (II) wherein R₁ is H, R₂, R₃, R₄, are independently selected from C₁-C₄ straight chain alkyl and x is 10 to 18, and wherein R₅ is unsubstituted benzyl and R₆ is C₁₂-C₁₅ alkyl.

Suitable emollients of the types indicated above include but are not limited to methyl isostearate, isopropyl isostearate, C12-15 alkyl benzoate, isostearyl neopentanoate.

A further water-insoluble, skin/hair care ingredient suitable for use herein is a vegetable oil adduct prepared from vegetable oils containing non-conjugated polyunsaturated fatty acid esters which are conjugated and elaidinized then modified by Diels-Alder addition with a member of the group consisting of acrylic acid, fumaric acid and maleic anhydride. The adducts and their preparation are described in US-A-4740367.

Preferred vegetable oils adducts for use herein can be generally described as those having the formula (I): 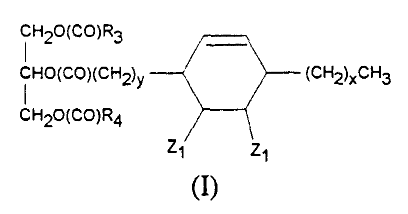 wherein x, y are integers of from 3 to 9, R₃ and R₄ are independently selected from saturated and unsaturated C₇-C₂₂ hydrocarbyl, each Z₁ being CO₂M and wherein M is H, alkali metal, alkaline earth metal, ammonium or alkanolammonium.

Preferred vegetable oil adducts for use herein are those of formula (I) wherein x+y= 12 (soybean oil) and adducts derived by Diels Alder addition of vegetable oils with maleic anhydride. Particularly preferred for use herein is maleated soybean oil which is commercially available under the tradename Ceraphyl GA from Van Dyke.

A particularly preferred water-insoluble hair/skin care ingredient for use herein is a vegetable triglyceride, especially soyabean oil.

### Optional Ingredients

### Polymeric Cationic Conditioning Agent

The compositions according to the present invention can optionally include a polymeric cationic conditioning agent. Any polymeric cationic conditioning agents are suitable for use in the compositions herein provided that they do raise the viscosity of the foaming composition to greater than 700 mPa.s. The polymeric skin conditioning agent is preferably present at a level from 0.01% to 5%, preferably from 0.01% to 3% and especially from 0.01% to 2% by weight.

Suitable polymers are high molecular weight materials (mass-average molecular weight determined, for instance, by light scattering, being generally from 2,000 to 5,000,000, preferably from 5,000 to 3,000,000 more preferably from 100,000 to 1,000,000).

Representative classes of polymers include cationic polysaccharides; cationic homopolymers and copolymers derived from acrylic and/or methacrylic acid; cationic cellulose resins; cationic copolymers of dimethyldiallylammonium chloride and acrylamide and or acrylic acid; cationic homopolymers of dimethyldiallylammonium chloride; cationic polyalkylene and ethoxypolyalkylene imines; quaternized silicones, and mixtures thereof. By way of exemplification, cationic polymers suitable for use herein include cationic guar gums such as hydroxypropyl trimethyl ammonium guar gum (d.s. of from 0.11 to 0.22) available commercially under the trade names Jaguar C-14-S(RTM) and Jaguar C-17(RTM) and also Jaguar C-16(RTM), which contains hydroxypropyl substituents (d.s. of from 0.8-1.1) in addition to the above-specified cationic groups, and quaternized hydroxy ethyl cellulose ethers available commercially under the trade names Ucare Polymer JR-30M, JR-400, Catanal (RTM) and Celquat. Other suitable cationic polymers are homopolymers of dimethyldiallylammonium chloride available commercially under the trade name Merquat 100, copolymers of dimethyl aminoethylmethacrylate and acrylamide, copolymers of dimethyldiallylammonium chloride and acrylamide, available commercially under the trade names Merquat 550 and Merquat S, acrylic acid/dimethyldiallylammonium chloride/acrylamide copolymers available under the trade name Merquat 3330, quaternized vinyl pyrrolidone acrylate or methacrylate copolymers of amino alcohol available commercially under the trade name Gafquat, for example Polyquaternium 11, 23 and 28 (quaternized copolymers of vinyl pyrrolidone and dimethyl aminoethylmethacrylate - Gafquat 755N and co-polymers of vinyl pyrrolidene and dimethylaminoethyl methacrylamide - HS-100), vinyl pyrrolidone/vinyl imidazolium methochloride copolymers available under the trade names Luviquat FC370, Polyquaternium 2, and polyalkyleneimines such as polyethylenimine and ethoxylated polyethylenimine.

In addition to the water-insoluble skin/hair care ingredient as described above the compositions of the invention can also contain from 0.1% to 20%, preferably from 1% to 15%, and more preferably from 2% to 10% by weight of an oil derived nonionic surfactant or mixture of oil derived nonionic surfactants. Oil derived nonionic surfactants are valuable in compositions according to the invention for the provision of skin feel benefits both in use and after use. Suitable oil derived nonionic surfactants for use herein include water soluble vegetable and animal-derived emollients such as triglycerides with a polyethyleneglycol chain inserted; ethoxylated mono and di-glycerides, polyethoxylated lanolins and ethoxylated butter derivatives. One preferred class of oil-derived nonionic surfactants for use herein have the general formula (XII) 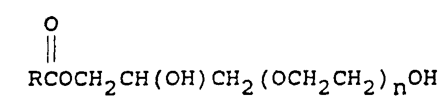 wherein n is from 5 to 200, preferably from 20 to 100, more preferably from 30 to 85, and wherein R comprises an aliphatic radical having on average from 5 to 20 carbon atoms, preferably from 7 to 18 carbon atoms.

Suitable ethoxylated oils and fats of this class include polyethyleneglycol derivatives of glyceryl cocoate, glyceryl caproate, glyceryl caprylate, glyceryl tallowate, glyceryl palmate, glyceryl stearate, glyceryl laurate, glyceryl oleate, glyceryl ricinoleate, and glyceryl fatty esters derived from triglycerides, such as palm oil, almond oil, and corn oil, preferably glyceryl tallowate and glyceryl cocoate.

Suitable oil derived nonionic surfactants of this class are available from Croda Inc. (New York, USA) under their Crovol line of materials such as Crovol EP40 (PEG 20 evening primrose glyceride), Crovol EP 70 (PEG 60 evening primrose glyceride) Crovol A-40 (PEG 20 almond glyceride), Crovol A-70 (PEG 60 almond glyceride), Crovol M-40 (PEG 20 maize glyceride), Crovol M-70 (PEG 60 maize glyceride), Crovol PK-40 (PEG 12 palm kernel glyceride), and Crovol PK-70 (PEG 45 palm kernel glyceride) and under their Solan range of materials such as Solan E, E50 and X polyethoxylated lanolins and Aqualose L-20 (PEG 24 lanolin alcohol) and Aqualose W15 (PEG 15 lanolin alcohol) available from Westbrook Lanolin. Further suitable surfactants of this class are commercially available from Sherex Chemical Co. (Dublin, Ohio, USA) under their Varonic LI line of surfactants and from Rewo under their Rewoderm line of surfactants.

These include, for example, Varonic LI 48 (polyethylene glycol (n=80) glyceryl tallowate, alternatively referred to as PEG 80 glyceryl tallowate), Varonic LI 2 (PEG 28 glyceryl tallowate), Varonic LI 420 (PEG 200 glyceryl tallowate), and Varonic LI 63 and 67 (PEG 30 and PEG 80 glyceryl cocoates), Rewoderm LI5-20 (PEG-200 palmitate), Rewoderm LIS-80 (PEG-200 palmitate with PEG-7 glyceryl cocoate) and Rewoderm LIS-75 (PEG-200 palmitate with PEG-7 glyceryl cocoate) and mixtures thereof. Other oil-derived emollients suitable for use are PEG derivatives of corn, avocado, and babassu oil, as well as Softigen 767 (PEG(6) caprylic/capric glycerides).

Also suitable for use herein are nonionic surfactants derived from composite vegetable fats extracted from the fruit of the Shea Tree (Butyrospermum Karkii Kotschy) and derivatives thereof. This vegetable fat, known as Shea Butter is widely used in Central Africa for a variety of means such as soap making and as a barrier cream, it is marketed by Sederma (78610 Le Perray En Yvelines, France). Particularly suitable are ethoxylated derivatives of Shea butter available from Karlshamn Chemical Co. (Columbos, Ohio, USA) under their Lipex range of chemicals, such as Lipex 102 E-75 and Lipex 102 E-3 (ethoxylated mono, di-glycerides of Shea butter) and from Croda Inc. (New York, USA) under their Crovol line of materials such as Crovol SB-70 (ethoxylated mono, di-glycerides of Shea butter). Similarly, ethoxylated derivatives of Mango, Cocoa and Illipe butter may be used in compositions according to the invention. Although these are classified as ethoxylated nonionic surfactants it is understood that a certain proportion may remain as non-ethoxylated vegetable oil or fat.

Other suitable oil-derived nonionic surfactants include ethoxylated derivatives of almond oil, peanut oil, rice bran oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazelnut oil, olive oil, grapeseed oil, and sunflower seed oil.

Oil derived nonionic surfactants highly preferred for use herein from the viewpoint of optimum mildness and skin feel characteristics are Lipex 102-3 (RTM) (PEG-3 ethoxylated derivatives of Shea Butter) and Softigen 767 (RTM) (PEG-6 caprylic/capric glycerides).

The compositions according to the present invention can also comprise lipophilic emulsifiers as skin care actives. Suitable lipophilic skin care actives include anionic food grade emulsifiers which comprise a di-acid mixed with a monoglyceride such as succinylated monoglycerides, monostearyl citrate, glyceryl monostearate diacetyl tartrate and mixtures thereof.

A preferred component of the foaming compositions herein is a non-aqueous, viscosity reducing, organic solvent. The term "solvent" is used herein to connote non-surface active or low surface active materials that dissolve into the detergent composition matrix having a viscosity reduction effect on the composition. This effect is generally a result of their interaction with the surfactant-water system present in the formulations preventing the formulation of liquid crystal phases. The term "solvent" is not meant to require that the solvent material be capable of actually dissolving all of the detergent composition components added thereto.

The non-aqueous organic materials which are employed as solvents herein can be liquids of high or low polarity. High polarity liquids suitable as solvents for use herein are for example short chain alcohols (ethanol, propanol, propane-diol, etc), short chain aldehydes (methylal, acetaldehyde, etc.), short chain ketones (acetone, propanone, etc) and short chain ethers.

Other polar materials useful in some cases are glycerols, glycols and short chain ethoxylated alcohols (short chain nonionic surfactants).

The short chain nonionic surfactants for use herein are alkoxylated alcohols according to the formula:

RO(A)nH,

wherein R is a C6 to C 10 straight or branched, hydrocarbon chain and n, representing the average ethoxylation degree, is from 1 to 10, or mixtures thereof. A is ethylene oxide or propylene oxide or mixtures thereof.

Suitable surfactants of the above type for use herein can readily be made by condensing alcohols having the desired chain length with propylene or ethylene oxide, or mixtures thereof. Suitable short chain alkoxylated alcohols for use herein are commercially available from several suppliers, for example, Dehydrol 04 from Henkel (C8EO4), Mergital C4 from Sidobre (C8EO4) and Imbentin AG/810/050 and AG/810/080 from Kolb (respectively C8-10EO5 and C8-10E08).

Suitable types of low-polarity solvents useful in the nonaqueous liquid detergent compositions herein include alkylene glycol mono lower alkyl ethers, lower molecular weight polyethylene glycols, lower molecular weight methyl esters and amides, and the like. A preferred type of nonaqueous, low-polarity solvent for use herein comprises the mono-, di-, tri-, or tetra-C₂-C₃ alkylene glycol mono C₂-C₆ alkyl ethers. The specific examples of such compounds include diethylene glycol monobutyl ether, tetraethylene glycol monobutyl ether, dipropolyene glycol monoethyl ether, and dipropylene glycol monobutyl ether. Diethylene glycol monobutyl ether and dipropylene glycol monobutyl ether are especially preferred. Compounds of the type have been commercially marketed under the tradenames Dowanol, Carbitol, and Cellosolve.

Another preferred type of nonaqueous, low-polarity organic solvent useful herein comprises the lower molecular weight polyethylene glycols (PEGs). Such materials are those having molecular weights of at least 150. PEGs of molecular weight ranging from 200 to 600 are most preferred.

Yet another preferred type of non-polar, nonaqueous solvent comprises lower molecular weight methyl esters. Such materials are those of the general formula : R¹-C(O)-OCH₃ wherein R¹ ranges from 1 to 18. Examples of suitable lower molecular weight methyl esters include methyl acetate, methyl propionate, methyl octanoate, and methyl dodecanoate.

The nonaqueous, organic solvent(s) employed should, of course, be compatible and non-reactive with other composition components, used in the foaming compositions herein. Such a solvent component will generally be utilized in an amount of from 1 % to 60% by weight of the composition. More preferably, the nonaqueous, organic solvent will comprise from 5% to 50% by weight of the composition.

Foam stabilising agents may also be employed in the compositions of the present invention. Especially preferred are alyphatic alcohols such as straight chain saturated alcohols of 12 to 18 carbon atoms e.g. cetyl alcohol, stearyl alcohol, myristyl alcohol and mixtures thereof. Polymers including polyvinylpyrrolidone, polyvinyl alcohol, polyacrylamide, polypeptides, polysaccharides, cellulose derivatives; and also natural and synthetic gums and resins such as guar gum, xanthan gum, carageenan, sodium alginate and caseinate may also be used in the present invention.

The cleansing compositions can optionally include other hair or skin moisturizers which are soluble in the cleansing composition matrix. The preferred level of such moisturizers is from 0.5% to 20% by weight. In preferred embodiments, the moisturizer is selected from essential amino acid compounds found naturally occurring in the stratum corneum of the skin and water-soluble nonpolyol nonocclusives and mixtures thereof.

Some examples of nonocclusive moisturizers are polybutene, squalane, sodium pyrrolidone carboxylic acid, D-panthenol, lactic acid, L-proline, guanidine, pyrrolidone, hydrolyzed protein and other collagen-derived proteins, aloe vera gel, acetamide MEA and lactamide MEA and mixtures thereof.

A number of additional optional materials can be added to the cleansing compositions each at a level of from 0.1% to 2% by weight. Such materials include water-compatible silicones, proteins and polypeptides and derivatives thereof; water-soluble or solubilizable preservatives, when water is present, includes Germall 115, methyl, ethyl, propyl and butyl esters of hydroxybenzoic acid, EDTA, Euxyl (RTM) K400, natural preservatives such as benzyl alcohol, potassium sorbate and bisabalol; sodium benzoate and 2-phenoxyethanol; other moisturizing agents such as hyaluronic acid, chitin , and starch-grafted sodium polyacrylates such as Sanwet (RTM) IM-1000, IM-1500 and IM-2500 available from Celanese Superabsorbent Materials, Portsmith, VA, USA and described in US-A-4,076,663; solvents ; suitable anti-bacterial agents; colouring agents; TiO₂ and TiO₂-coated mica; perfumes and perfume solubilizers; and zeolites such as Valfour BV400 and derivatives thereof and Ca²⁺/Mg²⁺ sequestrants such as polycarboxylates, amino polycarboxylates, polyphosphonates, amino polyphosphonates, EDTA etc, water softening agents such as sodium citrate and insoluble particulates such as zinc stearate and fumed silica.

The pH of the compositions is preferably from 3.5 to 10, more preferably from 6 to 9, especially from 5 to 8 and most preferably from 5 to 7.5.

The foaming compositions may be in the form of a heterogeneous system, a true solution or an oil-in-non-aqueous solvent emulsion.

### Propellant gas

The propellant gas of the present invention comprises carbon dioxide, or nitrous oxide (especially N2O), or mixtures thereof. Most preferred is carbon dioxide. Low molecular weight hydrocarbons, such as propane, butane, pentane, hexane, may optionally be included provided that flammability requirements are not exceeded.

Various ways to pressurise the propellant gas are known in the art. For example the gas may be pressurised at the time of packing. The product may be physically separated from a compressed gas by a membrane such as rubber under tension. Alternatively a means for pressurising the gas subsequently by mechanical action may be provided (so-called "pump and spray" systems).

Various apparatus for delivering foams are described in US-A 5 364 031 issued on 15th November 1994 entitled "Foam Dispensing Nozzles and Dispensers Employing Said Nozzles".
Any nozzle or nozzle / valve assembly which provides a means for releasing the mixture of detergent ingredients from the container and provides a foam is suitable for use in the present invention. The Precision Valve Company (Valve Précision in France) supplies a range of nozzle assemblies for various applications including shaving foams and carpet cleaners under various trade names including City®, Montego®, Power Jet®, Vulcan® and Visco®. Nozzles which disperse the foam both horizontally and vertically (when the container is held upright) are available. Metering nozzles which dispense a predetermined amount of foam are also available and useful in the present invention. Metering valves are disclosed in WO9108965 (Precision Valve Co) and EP-A 616953 (3M Co).

### Container

The packaged product of the present invention comprises a sealed container, such as an essentially cylindrical bottle, having a dispensing means such as a nozzle. The container contains the composition and propellant gas. Suitable containers may be made from any material, especially aluminium, tin-plate, plastics including PET, OPP, PE or polyamide and including mixtures, laminates or other combinations of these. Foam is dispensed when the nozzle is activated and the detergent is released together with the propellant gas. The propellant gas expands to form many "bubbles" within the composition thereby creating the foam.

The dispensed foam may be applied with the hand or preferably with a personal cleansing implement such as a puff. Suitable personal cleansing implements for use with the product of the present invention include those are disclosed in the following patent documents which are incorporated herein by reference: US-A-5,144,744 to Campagnoli, issued September 8,1992, US-A-3,343,196 to Barnhouse, WO95/26671 to The Procter & Gamble Company, WO95/00116 to The Procter & Gamble Company and WO95/26670 to The Procter & Gamble Company.

Below are examples which illustrate the products of the present invention.

### Examples 1-IV

| | I/% | II/% | III/% | IV/% |
|---|---|---|---|---|
| MEA AS | 6 | 12 | 8 | 6 |
| MEA AE3S | 24 | 31 | 24 | 18 |
| NMG | 8 | 15 | 0 | 8 |
| Soyabean Oil | 40 | 15 | 15 | 30 |
| MSO | 0 | 0 | 3 | 6 |
| PEG 6 Cap. | 0 | 0 | 0 | 13 |
| Glyc. Perfume | 1.3 | 1.3 | 1.3 | 1.3 |
| Citric Acid | 0 | 0 | 0 | 0 |
| Propylene Glycol | 12.82 | 18.84 | 12.54 | 10.47 |
| PEG 200 | 6.1 | 4.3 | 34.25 | 5.81 |
| Water | to 100 | to 100 | to 100 | to 100 |

### Examples V-VIII

| | V/% | VI/% | VII/% | VIII/% |
|---|---|---|---|---|
| MEA AS | 6 | 8 | 8 | 12 |
| MEA AE3S | 18 | 24 | 24 | 31 |
| NMG | 8 | 0 | 4 | 15 |
| Soyabean Oil | 30 | 30 | 30 | 0 |
| MSO | 6 | 6 | 6 | 0 |
| PEG 6 Cap. | 0 | 0 | 0 | 0 |
| Glyc. Perfume | 1.3 | 1.3 | 1.3 | 1.3 |
| Citric Acid | 0 | 0 | 0 | 0 |
| Propylene Glycol | 10.47 | 12.54 | 13.07 | 18.84 |
| PEG 200 | 18.81 | 16.25 | 11.73 | 19.3 |
| Water | to 100 | to 100 | to 100 | to 100 |
| Glycerine | 0 | 0 | 0 | 0 |

### Examples IX-XII

| | IX/% | X/% | XI/% | XII/% |
|---|---|---|---|---|
| MEA AS | 6 | 8 | 11.51 | 12.39 |
| MEA AE3S | 18 | 24 | 28.7 | 30.88 |
| NMG | 8 | 0 | 14.23 | 15.31 |
| Soyabean Oil | 0 | 0 | 0 | 0 |
| MSO | 0 | 0 | 0 | 0 |
| PEG 6 Cap. | 13 | 0 | 14.68 | 15.8 |
| Glyc. Perfume | 1.3 | 1.3 | 0.37 | 0.4 |
| Citric Acid | 0 | 0 | 0.41 | 0.44 |
| Propylene Glycol | 10.47 | 12.54 | 20.23 | 21.69 |
| PEG 200 | 35.81 | 46.25 | 0 | 0 |
| Water | to 100 | to 100 | to 100 | to 100 |
| Glycerine | 6 | 6 | 0 | 0 |

### Examples XIII-XVI

| | XIII/% | XIV/% | XV/% | XVI/% |
|---|---|---|---|---|
| MEA AS | 15.2 | 14.71 | 14.38 | 16.63 |
| MEA AE3S | 37.9 | 36.76 | 11.55 | 16.62 |
| NMG | 0 | 18.18 | 6.27 | 0 |
| Soyabean Oil | 0 | 0 | 30.14 | 30.14 |
| MSO | 0 | 0 | 5.65 | 5.65 |
| PEG 6 Cap. | 19.39 | 0 | 12.81 | 12.81 |
| Glyc. Perfume | 0.4 | 0.4 | 1.3 | 1.3 |
| Citric Acid | 0.54 | 0 | 0 | 0 |
| Propylene Glycol | 23.07 | 22.58 | 10.99 | 13.03 |
| PEG 200 | 0 | 3.86 | 5.37 | 0 |
| Water | to 100 | to 100 | to 100 | to 100 |
| Glycerine | 0 | 0 | 0 | 0 |
| Ucare Polymer JR30M | 0 | 0 | 0.2 | 0.2 |

### Example XVII

| | XVII/% |
|---|---|
| MEA AS | 10.48 |
| MEA AE3S | 8.43 |
| NMG | 4.61 |
| Soyabean Oil | 30.14 |
| MSO | 5.65 |
| PEG 6 Cap. | 12.81 |
| Glyc. | |
| Perfume | 1.3 |
| Citric Acid | 0.6 |
| Propylene Glycol | 11.88 |
| PEG 200 | 0 |
| Water | to 100 |
| AE7 | 12.07 |
| Ucare Polymer JR30M | 0.2 |

In the above examples I-XIII the abbreviations used for the various ingredients are explained below:
- MEA AS: monoethanolamine salt of cocyl sulphate
- MEA AE3S: monoethanolamine salt of laureth-3 sulphate
- NMG: C12-C14 N-methyl Glucose Amide
- AE7: C13-C15 alcohol with 7 moles of ethylene oxide
- PEG 6 Cap Glyc: PEG 6 Capric/Caprylic glycerides
- PEG 200: polyethylene glycol 200
- Ucare Polymer JR 30M: quaternized hydroxyethyl cellulose ether
- MSO: Maleated Soybean Oil

The foaming compositions of examples I-VII were be made by mixing the ingredients together. Then each composition was packed into metal containers having a nominal capacity of 210 cubic centimetres. The cans were then filled with 125g of liquid detergent and then were pressurised with carbon dioxide while shaking, until it equilibrates to a can pressure of 10 bars of carbon dioxide at 20°C.

All the cans are fitted with a 3 x 1.0 mm diameter standard valve (Code No. 045380 supplied by Valve Precision), without a dip tube and a straight whipped cream nozzle. In order to expel the foam out of the can, the can must be in an inverted position.

## Claims

1. A packaged personal cleansing product comprising:
(i) a foaming composition, wherein the foaming composition comprises less than 25% by weight, preferably less than 15% by weight, of water, at least 18% by weight of surfactant, said surfactant being selected from anionic, nonionic, cationic, zwitterionic and amphoteric surfactants and mixtures thereof, and from 1% to 50% by weight of a water-insoluble skin or hair care ingredient;
(ii) propellant gas; and
(iii) a container for (i) and (ii);
characterised in that the propellant gas comprises carbon dioxide, nitrous oxides, or mixtures thereof and that the viscosity of the foaming composition, measured at 20°C and 1 atmosphere, using a Brookfield viscometer, No.2 Spindle at 60 rpm, is less than 700 mPa.s.

2. A packaged product according to Claim 1 wherein the foaming composition further comprises a viscosity reducing, organic solvent.

3. A packaged product according to Claim 2 wherein the viscosity reducing, organic solvent is selected from the group consisting of polyethylene glycol, short chain alkyl ethoxylates, glycerol, Carbowax, butyl diglycerol ether, and mixtures thereof.

4. A packaged product according to any of Claims 1 to 3 wherein the propellant gas is selected from carbon dioxide, nitrous oxides, and mixtures thereof.

5. A packaged product according to any of Claims 1 to 4 wherein the propellant gas further comprises an organic propellant.

6. A packaged product according to Claim 5 wherein the propellant gas further comprises propane, butane, pentane, hexane, or mixtures thereof.

7. A packaged product according to any of Claims 1 to 6 wherein the anionic surfactant is selected from alkyl sulphates, ethoxylated alkyl sulphates, alkyl glyceryl ether sulfonates, methyl acyl taurates, fatty acyl glycinates, N-acyl glutamates, acyl isethionates, acyl ethoxylated isethionates, acyl short chain branched isethionates, paraffin sulphonates, alpha-olefin sulphonates, fatty amido ethoxylated carboxylates, alkyl sulfosuccinates, alkyl ethoxy sulphosuccinates, alpha-sulfonated fatty acids, their salts and/or their esters, alkyl phosphate esters, ethoxylated alkyl phosphate esters, acyl sarcosinates and fatty acid/protein condensates, and mixtures thereof.

8. A packaged product according to any of Claims 1 to 7 wherein the nonionic surfactant is selected from C12-C18 fatty acid mono- and di-ethanolamides, and ethoxylated derivatives thereof, ethoxylated fatty alcohols, alkyl poly glucosides, sucrose polyester surfactants and polyhydroxy fatty acid amide surfactants.

9. A packaged product according to any of Claims 1 to 8 wherein the amphoteric surfactant is selected from:
(a) imidazolinium derivatives of formula [IV] 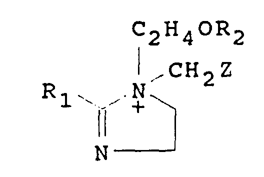 wherein R₁ is C₇-C₂₂ alkyl or alkenyl, R₂ is hydrogen of CH₂Z, each Z is independently CO₂ or CH₂ CO₂M, and M is H, alkali metal, alkaline earth metal, ammonium or alkanolammonium; and/or ammonium derivatives of formula [V] 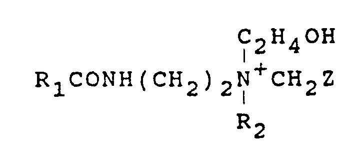 wherein R₁, R₂ and Z are as defined above:
(b) aminoalkanoates of formula [VI]
R₁NH(CH₂)ₙCO₂M
iminodialkanoates of formula [VII]
R₁N[(CH₂)ₘCO₂M]₂
and iminopolyalkanoates of formula (VIII) 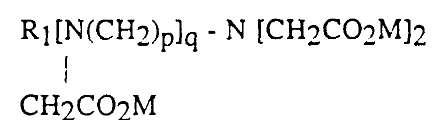 wherein n, m, p, and q are numbers from 1 to 4, and R₁ and M are independently selected from the groups specified above; and
(c) mixtures thereof.

10. A packaged composition according to any of Claims 1 to 9 wherein the zwitterionic surfactant is selected from alkyl betaine, amido betaine, alkylamido betaine, alkylamido sultaine, and mixtures thereof.

## Patentansprüche

1. Abgepacktes Reinigungsprodukt für den persönlichen Gebrauch, umfassend:
(i) eine schäumende Zusammensetzung, wobei die schäumende Zusammensetzung weniger als 25 Gew.-%, vorzugsweise weniger als 15 Gew.-%, Wasser und mindestens 18 Gew.-% Tensid umfaßt, wobei das Tensid aus anionischen, nichtionischen, kationischen, zwitterionschen und amphoteren Tensiden und Mischungen hiervon gewählt ist, und 1 bis 50 Gew.-% eines wasserunlöslichen Haut- oder Haarpflegemittelbestandteils;
(ii) Treibmittelgas; und
(iii) einen Behälter für (i) und (ii);
dadurch gekennzeichnet, daß das Treibmittelgas Kohlenstoffdioxid, Stickstoffoxide oder Mischungen hiervon umfaßt, und daß die Viskosität der schäumenden Zusammensetzung, gemessen bei 20°C und 1 Atmosphäre, unter Verwendung eines Brookfield-Viskosimeters, Spindel Nr. 2 bei 60 U/min weniger als 700 mPa.s beträgt.

2. Abgepacktes Produkt nach Anspruch 1, wobei die schäumende Zusammensetzung weiterhin ein die Viskosität reduzierendes, organisches Lösungsmittel umfaßt.

3. Abgepacktes Produkt nach Anspruch 2, wobei das die Viskosität reduzierende, organische Lösungsmittel gewählt ist aus der Gruppe, bestehend aus Polyethylenglykol, kurzkettigen Alkylethoxylaten, Glycerin, Carbowax, Butyldiglycerolether und Mischungen hiervon.

4. Abgepacktes Produkt nach mindestens einem der Ansprüche 1 bis 3, wobei das Treibmittelgas gewählt ist aus Kohlenstoffdioxid, Stickstoffoxiden und Mischungen hiervon.

5. Abgepacktes Produkt nach mindestens einem der Ansprüche 1 bis 4, wobei das Treibmittelgas weiterhin ein organisches Treibmittel umfaßt.

6. Abgepacktes Produkt nach Anspruch 5, wobei das Treibmittelgas weiterhin Propan, Butan, Pentan, Hexan oder Mischungen hiervon umfaßt.

7. Abgepacktes Produkt nach mindestens einem der Ansprüche 1 bis 6, wobei das anionische Tensid gewählt ist aus Alkylsulfaten, ethoxylierten Alkylsulfaten, Alkylglycerylethersulfonaten, Methylacyltauraten, Fettsäureacylglycinaten, N-Acylglutamaten, Acylisethionaten, acylethoxylierten Isethionaten, Acyl-kurzkettigen, verzweigten Isethionaten, Paraffinsulfonaten, alphaOlefinsulfonaten, Fettamido-ethoxylierten Carboxylaten, Alkylsulfosuccinaten, Alkylethoxysulfosuccinaten, alpha-sulfonierten Fettsäuren, deren Salzen und/ oder deren Estern, Alkylphosphatestern, ethoxylierten Alkylphosphatestern, Acylsarcosinaten und Fettsäure/Protein-Kondensaten, und Mischungen hiervon.

8. Abgepacktes Produkt nach mindestens einem der Ansprüche 1 bis 7, wobei das nichtionische Tensid gewählt ist aus C₁₂-C₁₈ Fettsäuremono- und diethanolamiden und ethoxylierten Derivaten hiervon, ethoxylierten Fettalkoholen, Alkylpolyglucosiden, Saccharosepolyester-Tensiden und Polyhydroxyfettsäureamid-Tensiden.

9. Abgepacktes Produkt nach mindestens einem der Ansprüche 1 bis 8, wobei das amphotere Tensid gewählt ist aus:
(a) Imidazolinium-Derivaten der Formal (IV) 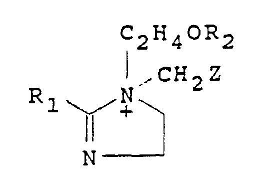 worin R₁ C₇-C₂₂-Alkyl oder -Alkenyl ist, R₂ Wasserstoff oder CH₂Z ist, jedes Z unabhängig CO₂ oder CH₂CO₂M ist, und M H, Alkalimetall, Erdalkalimetall, Ammonium oder Alkanolammonium ist; und/oder Ammoniumderivaten der Formel (V) 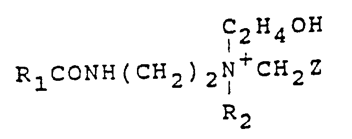 worin R₁, R₂ und Z wie oben definiert sind;
(b) Aminoalkanoaten der Formal (VI)
R₁NH(CH₂)ₙCO₂M
Iminodialkanoaten der Formel (VII)
R₁N[(CH₂)ₘCO₂M]₂
und Iminopolyalkanoaten der Formel (VIII) 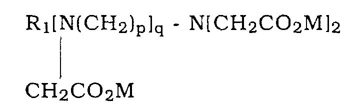 worin n, m, p und q Zahlen von 1 bis 4 sind, und R₁ und M unabhängig voneinander gewählt sind aus den oben angegebenen Gruppen; und
(c) Mischungen hiervon.

10. Abgepackte Zusammensetzung nach mindestens einem der Ansprüche 1 bis 9, wobei das zwitterionische Tensid gewählt ist aus Alkylbetain, Amidobetain, Alkylamidobetain, Alkylamidosultain und Mischungen hiervon.

## Revendications

1. Produit nettoyant personnel conditionné, comprenant :
(i) une composition moussante, la composition moussante comprenant moins de 25 % en poids, de préférence moins de 15 % en poids, d'eau, au moins 18% en poids d'agent tensioactif, ledit agent tensioactif étant choisi parmi les agents tensioactifs anioniques, non ioniques, cationiques, zwittérioniques et amphotères et des mélanges de ceux-ci, et 1 % à 50 % en poids d'un ingrédient pour le soin de la peau ou des cheveux insoluble dans l'eau ;
(ii) un gaz propulseur ; et
(iii) un récipient pour (i) et (ii) ;
caractérisé en ce que le gaz propulseur comprend du dioxyde de carbone, des oxydes nitreux ou des mélanges de ceux-ci et en ce que la viscosité de la composition moussante, mesurée à 20 °C et 1 atmosphère, en utilisant un viscomètre Brookfield, vis n° 2 à 60 tr/min, est inférieure à 700 mPa.s.

2. Produit conditionné selon la revendication 1, dans lequel la composition moussante comprend en outre un solvant organique réduisant la viscosité.

3. Produit conditionné selon la revendication 2, dans lequel le solvant organique réduisant la viscosité est choisi dans le groupe constitué par un polyéthylèneglycol, les éthoxylates d'alkyle à chaîne courte, le glycérol, le Carbowax, l'éther butylique de diglycérol et des mélanges de ceux-ci.

4. Produit conditionné selon l'une quelconque des revendications 1 à 3, dans lequel le gaz propulseur est choisi parmi le dioxyde de carbone, les oxydes nitreux et des mélanges de ceux-ci.

5. Produit conditionné selon l'une quelconque des revendications 1 à 4, dans lequel le gaz propulseur comprend en outre un propulseur organique.

6. Produit conditionné selon la revendication 5, dans lequel le gaz propulseur comprend en outre du propane, du butane, du pentane, de l'hexane ou des mélanges de ceux-ci.

7. Produit conditionné selon l'une quelconque des revendications 1 à 6, dans lequel l'agent tensioactif anionique est choisi parmi les sulfates d'alkyle, les sulfates d'alkyle éthoxylés, les sulfonates d'éther d'alkyle et de glycéryle, les taurates de méthyle et d'acyle, les glycinates d'acyle gras, les glutamates de N-acyle, les iséthionates d'acyle, les iséthionates d'acyle éthoxylés, les iséthionates d'acyle ramifiés à chaîne courte, les sulfonates de paraffine, les sulfonates d'alpha-oléfine, les amidocarboxylates éthoxylés gras, les sulfosuccinates d'alkyle, les éthoxy sulfosuccinates d'alkyle, les acides gras alpha-sulfonés, leurs sels et/ou leurs esters, les esters phosphates d'alkyle, les esters phosphates d'alkyle éthoxylés, les sarcosinates d'acyle et les condensats acide gras/protéine, et des mélanges de ceux-ci.

8. Produit conditionné selon l'une quelconque des revendications 1 à 7, dans lequel l'agent tensioactif non ionique est choisi parmi les mono- et di-éthanolamides d'acide gras en C₁₂ à C₁₈, et les dérivés éthoxylés de ceux-ci, les alcools gras éthoxylés, les alkylpolyglucosides, les agents tensioactifs polyesters de saccharose et les agents tensioactifs amides d'acide gras polyhydroxylés.

9. Produit conditionné selon l'une quelconque des revendications 1 à 8, dans lequel l'agent tensioactif amphotère est choisi parmi :
(a) les dérivés d'imidazolinium de formule [IV] 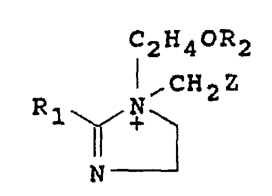 dans laquelle R₁ est un groupe alkyle ou alcényle en C₇ à C₂₂, R₂ est de l'hydrogène ou CH₂Z, chaque Z est indépendamment CO₂ ou CH₂CO₂M, et M est H, un métal alcalin, un métal alcalino-terreux, un groupe ammonium ou alcanolammonium ; et/ou des dérivés d'ammonium de formule [V] 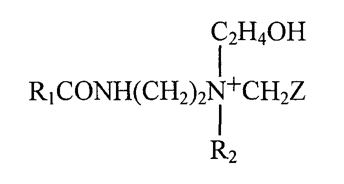 dans laquelle R₁, R₂ et Z sont tels que définis ci-dessus ;
(b) les aminoalcanoates de formule [VI]
R₁NH(CH₂)ₙCO₂M
les iminodialcanoates de formule [VII]
R₁N[(CH₂)ₘCO₂M]₂
et les iminopolyalcanoates de formule (VIII) 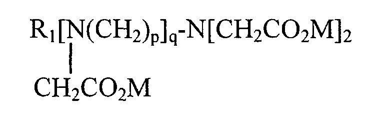 dans lesquelles n, m, p et q sont des nombres de 1 à 4, et R₁ et M sont indépendamment choisis dans les groupes spécifiés ci-dessus ; et
(c) des mélanges de ceux-ci.

10. Composition conditionnée selon l'une quelconque des revendications 1 à 9, dans laquelle l'agent tensioactif zwittérionique est choisi parmi une alkylbétaïne, une amidobétaïne, une alkylamidobétaïne, une alkylamidosultaïne et des mélanges de celles-ci.
